# EUROPÄISCHE PATENTSCHRIFT

(11) **EP 3 427 635 B1**
(45) Veröffentlichungstag und Bekanntmachung des Hinweises auf die Patenterteilung: **04.08.2021**
(21) Anmeldenummer: 18177494.4
(22) Anmeldetag: 13.06.2018
(51) Int. Cl.: A61B 1/015, A61M 1/00, A61B 1/12, A61B 17/00, A61B 1/307, A61M 39/22

(54) **MEDIZINISCHES INSTRUMENT**
MEDICAL INSTRUMENT
INSTRUMENT MÉDICAL

(30) Priorität: 10.07.2017 DE 102017115379
(43) Veröffentlichungstag der Anmeldung: 16.01.2019
(73) Patentinhaber: Karl Storz SE & Co. KG, 78532 Tuttlingen (DE)
(72) Erfinder: Löffler, Oliver, 78532 Tuttlingen (DE); Wittke, Uwe, 78532 Tuttlingen (DE); Rehbein, Stefan, 78532 Tuttlingen (DE); Doll, Frank, 78532 Tuttlingen (DE); Hermle, Rainer, 78532 Tuttlingen (DE); Nagele, Udo, 6300 Wörgl (AT)

(56) Entgegenhaltungen:
- DE-A1-102007 003 690
- US-A- 5 005 604
- US-A1- 2012 010 464
- US-A1- 2012 271 110

## Beschreibung

Die vorliegende Erfindung ist auf ein Resektoskop oder ein Zystoskop oder ein Hysteroskop oder ein anderes medizinisches Instrument für diagnostische, therapeutische und/oder chirurgische Maßnahmen in einem Hohlraum im Körper eines Patienten mit einer Zuleitungseinrichtung zum Zuführen eines Fluids zu dem Hohlraum und einer Ableitungseinrichtung zum Ableiten eines Fluids aus dem Hohlraum bezogen.

Ein Resektoskop, ein Zystoskop, ein Hysteroskop oder ein anderes medizinisches Instrument für diagnostische, therapeutische und/oder chirurgische Maßnahmen in einem Hohlorgan oder in einem anderen natürlichen oder künstlichen Hohlraum im Körper eines Patienten weist in der Regel Fluidkanäle zum Zuführen eines Spülfluids und zum Ableiten des Spülfluids aus dem Hohlraum auf. In einem proximalen Bereich eines solchen medizinischen Instruments sind zwei Ventile vorgesehen, mittels derer einerseits der Zufluss des frischen Spülfluids und andererseits der Abfluss von Fluid aus dem Hohlraum gesteuert werden können. Zum Füllen und/oder Erweitern des Hohlraums wird das Ventil im Zufluss geöffnet und das Ventil im Abfluss geschlossen. Danach justiert das medizinische Personal die Ventile manuell so, dass sich ein dynamisches Gleichgewicht einstellt. Der Füllgrad und der Druck im Hohlraum, die sich dabei einstellen, ist von der Justage der beiden Ventile und damit von der Erfahrung und der Aufmerksamkeit des medizinischen Personals abhängig. Fehler des medizinischen Personals können insbesondere einen zu hohen Druck in dem Hohlraum und damit eine Schädigung des Patienten zur Folge haben.

Die Patentanmeldung US 2012/0271110 A1 zeigt ein Endoskop mit kontinuierlicher Spülung, bei der Strömungswiderstand der Zuleitungsströmung größer ist als der Strömungswiderstand der Ableitungsströmung.

Das Patent US 5,005,604 zeigt ein Messgerät zum Steuern intravenöser Fluide mit einem Ventil, das Zu- und Ablauf von Fluiden, also insb. intravenösen Fluiden, steuern lässt, insb. stufenlos steuern lässt. Eine Aufgabe der vorliegenden Erfindung besteht darin, ein verbessertes medizinisches Instrument zu schaffen, das insbesondere eine höhere Sicherheit gegen Fehlbedienung bietet.

Diese Aufgabe wird durch den Gegenstand des unabhängigen Anspruchs gelöst. Weiterbildungen sind in den abhängigen Ansprüchen angegeben.

Ein medizinisches Instrument für diagnostische, therapeutische oder chirurgische Maßnahmen in einem Hohlraum im Körper eines Patienten umfasst einen proximalen Bereich, der zur Anordnung außerhalb des Körpers vorgesehen und ausgebildet ist, einen distalen Bereich, der zur Anordnung innerhalb eines Hohlraums in dem Körper vorgesehen und ausgebildet ist, eine Zuleitungseinrichtung zum Zuführen eines Fluids zu dem Hohlraum, wobei die Zuleitungseinrichtung von einer ersten Kupplung in dem proximalen Bereich des medizinischen Instruments bis zu einem Auslass in dem distalen Bereich reicht, und eine Ableitungseinrichtung zum Ableiten eines Fluids aus dem Hohlraum, wobei die Ableitungseinrichtung von einem Einlass in dem distalen Bereich des medizinischen Instruments bis zu einer zweiten Kupplung in dem proximalen Bereich des medizinischen Instruments reicht, wobei der Strömungswiderstand der Zuleitungseinrichtung größer ist als der Strömungswiderstand der Ableitungseinrichtung.

Das medizinische Instrument ist insbesondere für diagnostische, therapeutische und/oder chirurgische Maßnahmen in einem Hohlorgan oder in einem anderen natürlichen oder künstlich geschaffenen Hohlraum im Körper eines Patienten vorgesehen und ausgebildet.

Das medizinische Instrument ist insbesondere ein Resektoskop oder ein Zystoskop oder ein Hysteroskop. Der proximale Bereich des medizinischen Instruments kann ein Arbeitselement, das eine präzise manuelle Bewegung einer Lasersonde oder einer mono- oder bipolaren elektrochirurgischen Sonde ermöglicht, und/oder andere Einrichtungen zur manuellen Handhabung und Steuerung des medizinischen Instruments umfassen. Der distale Bereich des medizinischen Instruments wird insbesondere durch einen Schaft gebildet, innerhalb dessen ein großer Teil der Zuleitungseinrichtung und ein großer Teil der Ableitungseinrichtung vorgesehen sein können. Wenn der distale Bereich des medizinischen Instruments durch einen Schaft gebildet ist, können an oder nahe dessen distalem Ende der Auslass der Zuleitungseinrichtung und/oder der Einlass der Ableitungseinrichtung vorgesehen sein.

Die erste Kupplung und die zweite Kupplung umfassen jeweils insbesondere eine Luer-Kupplung oder eine Luer-Lock-Kupplung und/oder eine Schlauchkupplung.

Wenn das medizinische Instrument bei seiner vorgesehenen Verwendung mehrere verschiedene mögliche oder zumindest vorgesehene Konfigurationen aufweist, d. h. der Betrieb des medizinischen Instruments in mehreren verschiedenen Konfigurationen möglich oder vorgesehen ist, ist bei jeder möglichen oder bei jeder vorgesehenen Konfiguration des medizinischen Instruments der Strömungswiderstand der Zuleitungseinrichtung größer als der Strömungswiderstand der Ableitungseinrichtung.

Dass der Strömungswiderstand der Zuleitungseinrichtung größer ist als der Strömungswiderstand der Ableitungseinrichtung hat zur Folge, dass sich in dem Hohlraum ein Druck einstellt, der näher bei dem an der zweiten Kupplung vorliegenden Druck als bei dem an der ersten Kupplung vorliegenden Druck liegt. Der in dem Hohlraum vorliegende Druck ist damit in der Regel niedriger als der mit einem herkömmlichen medizinischen Instrument bei im Übrigen gleicher Konfiguration erzielbare maximale Druck. Das Risiko einer Schädigung eines Patienten durch einen zu hohen Druck in dem Hohlraum ist dadurch deutlich gesenkt.

Bei einem medizinischen Instrument, wie es hier beschrieben ist, ist insbesondere die Ableitungseinrichtung bei der vorgesehenen Verwendung des medizinischen Instruments nicht verschließbar oder nicht unbedingt verschließbar.

Ein medizinisches Instrument für diagnostische, therapeutische oder chirurgische Maßnahmen in einem Hohlraum im Körper eines Patienten umfasst einen proximalen Bereich, der zur Anordnung außerhalb des Körpers vorgesehen und ausgebildet ist, einen distalen Bereich, der zur Anordnung innerhalb eines Hohlraums in dem Körper vorgesehen und ausgebildet ist, eine Zuleitungseinrichtung zum Zuführen eines Fluids zu dem Hohlraum, wobei die Zuleitungseinrichtung von einer ersten Kupplung in dem proximalen Bereich des medizinischen Instruments bis zu einem Auslass in dem distalen Bereich reicht, und eine Ableitungseinrichtung zum Ableiten eines Fluids aus dem Hohlraum, wobei die Ableitungseinrichtung von einem Einlass in dem distalen Bereich des medizinischen Instruments bis zu einer zweiten Kupplung in dem proximalen Bereich des medizinischen Instruments reicht, wobei die Ableitungseinrichtung bei der vorgesehenen Verwendung des medizinischen Instruments nicht verschließbar oder nicht unbedingt verschließbar ist.

Die vorgesehene Verwendung umfasst insbesondere alle Konfigurationen und Szenarien, für die das medizinische Instrument zugelassen ist. Indem die Ableitungseinrichtung bei der vorgesehenen Verwendung nicht verschließbar ist, kann der Druck in dem Hohlraum nicht bis zu dem an der ersten Kupplung vorliegenden Druck ansteigen, sondern bleibt immer unter dem bei der ersten Kupplung vorliegenden Druck.

Die Ableitungseinrichtung ist dann nicht unbedingt verschließbar, wenn Sie nicht oder nur so verschließbar ist, dass sie nur unter bestimmten Bedingungen geschlossen ist oder geschlossen bleibt. Die Ableitungseinrichtung ist insbesondere dann nicht unbedingt verschließbar, wenn sie nur auf eine Weise verschließbar ist, die bei Überschreiten eines vorbestimmten Drucks ein Öffnen der Ableitungseinrichtung zur Folge hat.

Bei einem medizinischen Instrument, wie es hier beschrieben ist, umfasst die Ableitungseinrichtung insbesondere ein Sicherheitsventil, das bei Überschreiten eines vorbestimmten Drucks in der Ableitungseinrichtung öffnet.

Ein medizinisches Instrument für diagnostische, therapeutische oder chirurgische Maßnahmen in einem Hohlraum im Körper eines Patienten umfasst einen proximalen Bereich, der zur Anordnung außerhalb des Körpers vorgesehen und ausgebildet ist, einen distalen Bereich, der zur Anordnung innerhalb eines Hohlraums in dem Körper vorgesehen und ausgebildet ist, eine Zuleitungseinrichtung zum Zuführen eines Fluids zu dem Hohlraum, wobei die Zuleitungseinrichtung von einer ersten Kupplung in dem proximalen Bereich des medizinischen Instruments bis zu einem Auslass in dem distalen Bereich reicht, und eine Ableitungseinrichtung zum Ableiten eines Fluids aus dem Hohlraum, wobei die Ableitungseinrichtung von einem Einlass in dem distalen Bereich des medizinischen Instruments bis zu einer zweiten Kupplung in dem proximalen Bereich des medizinischen Instruments reicht, wobei die Ableitungseinrichtung ein Sicherheitsventil, das bei Überschreiten eines vorbestimmten Drucks in der Ableitungseinrichtung öffnet, umfasst.

Das Sicherheitsventil kann eine manuell oder auf andere Weise einstellbare Konfiguration aufweisen, bei der es unabhängig von dem in der Ableitungseinrichtung vorliegenden Druck offen ist. Alternativ kann das Sicherheitsventil ausgebildet sein, um ausschließlich abhängig von dem in der Ableitungseinrichtung vorliegenden Druck zu öffnen. Das Sicherheitsventil kann ferner ausgebildet sein, um bei Unterschreiten des vorbestimmten Drucks oder bei Unterschreiten eines weiteren, niedrigeren vorbestimmten Drucks zu schließen. Alternativ kann das Sicherheitsventil ausgebildet sein, um nach einem durch Überschreiten des vorbestimmten Drucks ausgelösten Öffnen nur durch manuelle oder andere Steuerung wieder geschlossen werden zu können.

Bei einem medizinischen Instrument, wie es hier beschrieben ist, weist insbesondere zumindest entweder die Zuleitungseinrichtung ein Strömungswiderstandsmittel, das den Strömungswiderstand der Zuleitungseinrichtung im Wesentlichen bestimmt, oder die Ableitungseinrichtung ein Strömungswiderstandsmittel, das den Strömungswiderstand der Ableitungseinrichtung im Wesentlichen bestimmt, auf.

Das Strömungswiderstandsmittel oder die Strömungswiderstandsmittel sind insbesondere in oder nahe dem proximalen Bereich des medizinischen Instruments angeordnet. Insbesondere ist ein Strömungswiderstandsmittel zwischen der ersten Kupplung und einer in einem Schaft des medizinischen Instruments angeordneten ersten Leitung, die Bestandteil der Zuleitungseinrichtung ist, angeordnet. Alternativ oder zusätzlich kann ein Strömungswiderstandsmittel zwischen einer in dem Schaft angeordneten zweiten Leitung, die Bestandteil der Ableitungseinrichtung ist, und der zweiten Kupplung angeordnet sein.

Wenn die Zuleitungseinrichtung ein Strömungswiderstandsmittel aufweist, beträgt bei den bei der vorgesehenen Verwendung vorliegenden Fluideigenschaften, Drücken und Flüssen der Strömungswiderstand dieses Strömungswiderstandsmittels insbesondere mindestens ein Zehntel oder mindestens ein Fünftel oder mindestens ein Viertel oder mindestens ein Drittel oder mindestens die Hälfte oder mindestens zwei Drittel oder mindestens drei Viertel oder mindestens vier Fünftel oder mindestens neun Zehntel des Strömungswiderstands der gesamten Zuleitungseinrichtung. Wenn die Ableitungseinrichtung ein Strömungswiderstandsmittel aufweist, beträgt bei den bei der vorgesehenen Verwendung vorliegenden Fluideigenschaften, Drücken und Flüssen der Strömungswiderstand dieses Strömungswiderstandsmittels insbesondere mindestens die Hälfte oder mindestens zwei Drittel oder mindestens drei Viertel oder mindestens vier Fünftel oder mindestens neun Zehntel des Strömungswiderstands der gesamten Ableitungseinrichtung. Wenn das medizinische Instrument mehrere verschiedene Konfigurationen aufweist, gilt dies insbesondere für jede mögliche oder zumindest für jede vorgesehene Konfiguration des medizinischen Instruments.

Bei einem medizinischen Instrument, wie es hier beschrieben ist, liegt insbesondere bei dem Strömungswiderstandsmittel die geringste Querschnittsfläche der Zuleitungseinrichtung oder der Ableitungseinrichtung vor.

Wenn die Zuleitungseinrichtung ein Strömungswiderstandsmittel aufweist, liegt insbesondere bei dem Strömungswiderstandsmittel die geringste Querschnittsfläche der Zuleitungseinrichtung vor. Wenn die Ableitungseinrichtung ein Strömungswiderstandsmittel aufweist, liegt insbesondere bei dem Strömungswiderstandsmittel die geringste Querschnittsfläche der Ableitungseinrichtung vor.

Bei einem medizinischen Instrument, wie es hier beschrieben ist, weist das Strömungswiderstandsmittel insbesondere einen einstellbaren Strömungswiderstand auf.

Der Strömungswiderstand des Strömungswiderstandsmittels kann beispielsweise durch Veränderung der Querschnittsfläche des Strömungswiderstandsmittels oder durch eine Veränderung der Konfiguration des Strömungswiderstandsmittels veränderbar sein. Unterschiedliche Werte des Strömungswiderstands des Strömungswiderstandsmittels haben unterschiedliche Drücke in einem Hohlraum, in dem mittels des medizinischen Instruments diagnostische, therapeutische oder chirurgische Maßnahmen ausgeführt werden, zur Folge.

Bei einem medizinischen Instrument, wie es hier beschrieben ist, weist das Strömungswiderstandsmittel insbesondere eine endliche Anzahl alternativer vorbestimmter Konfigurationen mit unterschiedlichen Strömungswiderständen auf, wobei das Strömungswiderstandsmittel vorgesehen und ausgebildet ist, um ausschließlich in den vorbestimmten Konfigurationen betrieben zu werden.

Ein medizinisches Instrument für diagnostische, therapeutische oder chirurgische Maßnahmen in einem Hohlraum im Körper eines Patienten umfasst einen proximalen Bereich, der zur Anordnung außerhalb des Körpers vorgesehen und ausgebildet ist, einen distalen Bereich, der zur Anordnung innerhalb eines Hohlraums in dem Körper vorgesehen und ausgebildet ist, eine Zuleitungseinrichtung zum Zuführen eines Fluids zu dem Hohlraum, wobei die Zuleitungseinrichtung von einer ersten Kupplung in dem proximalen Bereich des medizinischen Instruments bis zu einem Auslass in dem distalen Bereich reicht, eine Ableitungseinrichtung zum Ableiten eines Fluids aus dem Hohlraum, wobei die Ableitungseinrichtung von einem Einlass in dem distalen Bereich des medizinischen Instruments bis zu einer zweiten Kupplung in dem proximalen Bereich des medizinischen Instruments reicht, und ein Strömungswiderstandsmittel in der Zuleitungseinrichtung oder in der Ableitungseinrichtung, wobei das Strömungswiderstandsmittel eine endliche Anzahl alternativer vorbestimmter Konfigurationen mit unterschiedlichen Strömungswiderständen aufweist, und wobei das Strömungswiderstandsmittel vorgesehen und ausgebildet ist, um ausschließlich in den vorbestimmten Konfigurationen betrieben zu werden.

Das Strömungswiderstandsmittel weist insbesondere weniger als zehn, beispielsweise zwei, drei, vier oder fünf alternative vorbestimmte Konfigurationen mit unterschiedlichen Strömungswiderständen auf. Das Strömungswiderstandsmittel weist insbesondere mehrere verschiedene Kanäle oder Bohrungen mit unterschiedlichen Querschnitten und unterschiedlichen Strömungswiderständen auf, wobei in unterschiedlichen Konfigurationen des Strömungswiderstandsmittels Fluid durch unterschiedliche Kanäle oder Bohrungen fließt.

Bei einem medizinischen Instrument, wie es hier beschrieben ist, weist das Strömungswiderstandsmittel insbesondere eine Rasteinrichtung, die nur die vorbestimmten Konfigurationen des Strömungswiderstandsmittels zulässt oder die vorbestimmten Konfigurationen gegenüber anderen Konfigurationen bevorzugt, auf.

Die Rasteinrichtung umfasst beispielsweise eine elastisch auslenkbare Rastnase oder eine federbelastete Kugel, die in eine von mehreren Nuten oder anderen Ausnehmungen eingreifen kann. In jeder der vorbestimmten Konfigurationen des Strömungswiderstandsmittels greift die Rastnase oder die Kugel in eine der Nuten oder Ausnehmungen ein.

Alternativ kann die Rasteinrichtung beispielsweise einen oder mehrere Magneten und optional ein oder mehrere weichmagnetische Bauteile aufweisen. In diesem Fall liegen bei den vorbestimmten Konfigurationen jeweils zwei ungleichnamige Pole verschiedener Magneten einander gegenüber oder ein Pol eines Magneten weist einen minimalen Abstand von einer weichmagnetischen Einrichtung auf.

Bei einem medizinischen Instrument, wie es hier beschrieben ist, weist das Strömungswiderstandsmittel insbesondere ein rotierbares oder entlang eines geraden oder gekrümmten Pfads bewegbares Bauelement mit mehreren Durchgangsbohrungen auf, wobei in jeder vorbestimmten Konfiguration des Strömungswiderstandsmittels Fluid durch eine oder mehrere der Durchgangsbohrungen strömen kann.

Das Strömungswiderstandsmittel ist insbesondere ein Kükenventil mit einem konischen Küken, das um seine Symmetrieachse rotierbar ist und im Wesentlichen orthogonal zu seiner Symmetrieachse die Durchgangsbohrungen aufweist. Alternativ kann das bewegbar Bauelement beispielsweise als entlang eines geraden Pfads bewegbarer Schieber mit mehreren nebeneinander angeordneten Durchgangsbohrungen ausgebildet sein.

Bei einem medizinischen Instrument, wie es hier beschrieben ist, weisen die Durchgangsbohrungen insbesondere unterschiedliche Querschnitte auf, wobei in jeder vorbestimmten Konfiguration des Strömungswiderstandsmittels Fluid durch eine der Durchgangsbohrungen strömen kann.

Die Durchgangsbohrungen sind insbesondere in dem beschriebenen bewegbaren Bauelement so angeordnet, dass beim Bewegen des Bauelements während des Verschließens einer Durchgangsbohrung bereits eine benachbarte Durchgangsbohrung geöffnet wird. Diese Anordnung kann zur Folge haben, dass selbst bei allen Konfigurationen zwischen den vorbestimmten Konfigurationen das Strömungswiderstandsmittel nie ganz geschlossen ist.

Bei einem medizinischen Instrument, wie es hier beschrieben ist, weist die Ableitungseinrichtung insbesondere ein Strömungswiderstandsmittel auf, wobei das Strömungswiderstandsmittel der Ableitungseinrichtung in keiner möglichen oder in keiner vorgesehenen Konfiguration des medizinischen Instruments die Ableitungseinrichtung vollständig verschließt oder das Strömungswiderstandsmittel der Ableitungseinrichtung in keiner möglichen oder in keiner vorgesehenen Konfiguration des medizinischen Instruments die Ableitungseinrichtung unbedingt vollständig verschließt.

Das Strömungswiderstandsmittel der Ableitungseinrichtung verschließt die Ableitungseinrichtung beispielsweise dann nur bedingt, wenn es die Ableitungseinrichtung nur bei Vorliegen eines Drucks in der Ableitungseinrichtung, der unter einem vorbestimmten Schwellenwert liegt, verschließt.

Die vorgesehenen Konfigurationen des medizinischen Instruments sind insbesondere die vorgesehenen Konfigurationen des Strömungswiderstandsmittels der Ableitungseinrichtung. Die möglichen Konfigurationen des medizinischen Instruments sind insbesondere die möglichen Konfigurationen des Strömungswiderstandsmittels der Ableitungseinrichtung. Indem das Strömungswiderstandsmittel der Ableitungseinrichtung die Ableitungseinrichtung in keiner vorgesehenen Konfiguration und optional auch in keiner möglichen Konfiguration des medizinischen Instruments vollständig verschließt, bleibt der Druck in einem Hohlraum im Körper eines Patienten, in dem mittels des medizinischen Instruments eine diagnostische, therapeutische oder chirurgische Maßnahme vorgenommen wird, immer kleiner als der an der ersten Kupplung vorliegende Druck.

Bei einem medizinischen Instrument, wie es hier beschrieben ist, weist insbesondere sowohl die Zuleitungseinrichtung als auch die Ableitungseinrichtung jeweils ein Strömungswiderstandsmittel auf, wobei der Strömungswiderstand des Strömungswiderstandsmittels der Zuleitungseinrichtung größer ist als der Strömungswiderstand des Strömungswiderstandsmittels der Ableitungseinrichtung.

Insbesondere ist bei jeder vorgesehenen Konfiguration oder bei jeder möglichen Konfiguration des medizinischen Instruments der Strömungswiderstand des Strömungswiderstandsmittels der Zuleitungseinrichtung größer als der Strömungswiderstand des Strömungswiderstandsmittels der Ableitungseinrichtung.

Wenn der Strömungswiderstand des Strömungswiderstandsmittels der Zuleitungseinrichtung größer ist als der Strömungswiderstand des Strömungswiderstandsmittels der Ableitungseinrichtung und die Strömungswiderstände der übrigen Bestandteile der Zuleitungseinrichtung und der Ableitungseinrichtung gleich groß sind, stellt sich in dem Hohlraum ein Druck ein, der näher bei dem an der zweiten Kupplung vorliegenden Druck als bei dem an der ersten Kupplung vorliegenden Druck liegt. Dieser im Vergleich zu herkömmlichen medizinischen Instrumenten niedrigere Druck schont den Patienten.

Bei einem medizinischen Instrument, wie es hier beschrieben ist, ist insbesondere der minimale Querschnitt des Strömungswiderstandsmittels der Zuleitungseinrichtung kleiner als der minimale Querschnitt des Strömungswiderstandsmittels der Ableitungseinrichtung.

### Kurzbeschreibung der Figuren

Nachfolgend werden Ausführungsformen anhand der beigefügten Figuren näher erläutert. Es zeigen:
- Figur 1: eine schematische Darstellung eines medizinischen Instruments;
- Figur 2: eine schematische Darstellung eines weiteren medizinischen Instruments;
- Figur 3: eine schematische Darstellung eines Strömungswiderstandsmittels;
- Figur 4: eine schematische Darstellung eines weiteren Strömungswiderstandsmittels;
- Figur 5: eine schematische Darstellung einer Variante des Strömungswiderstandsmittels aus Figur 3;
- Figur 6: eine weitere schematische Darstellung des Strömungswiderstandsmittels aus Figur 5;
- Figur 7: eine schematische Darstellung einer Variante des Strömungswiderstandsmittels aus Figur 4;
- Figur 8: eine weitere schematische Darstellung des Strömungswiderstandsmittels aus Figur 7;
- Figur 9: eine schematische Darstellung eines weiteren Strömungswiderstandsmittels;
- Figur 10: eine weitere schematische Darstellung des Strömungswiderstandsmittels aus Figur 9.

### Beschreibung der Ausführungsformen

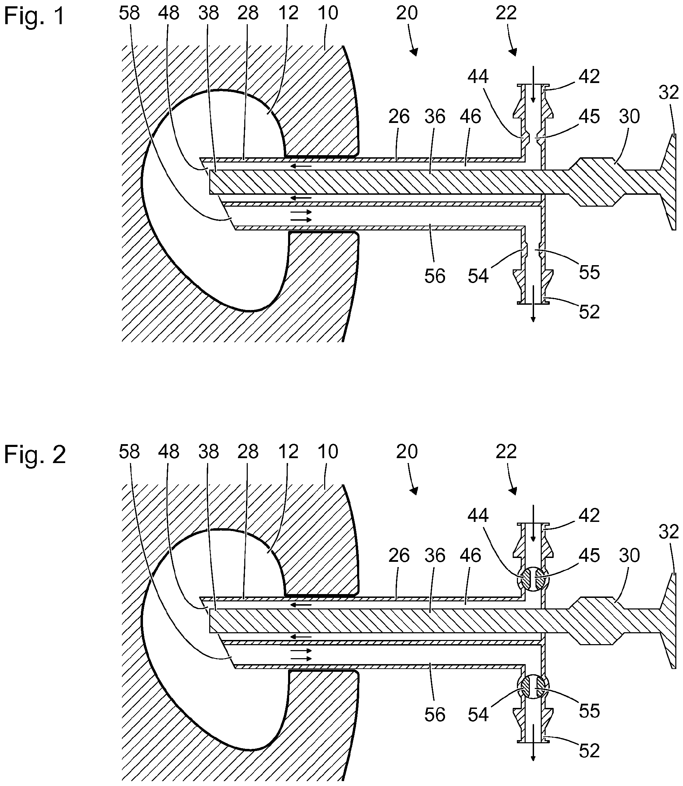

Figur 1 zeigt eine schematische Darstellung eines Schnitts durch einen Körper 10 eines Patienten und durch ein medizinisches Instrument 20. Die Schnittebene der Figur 1 enthält eine Längsachse des medizinischen Instruments 20.

Der Körper 10 weist einen Hohlraum 12 auf. Der Hohlraum 12 ist beispielsweise die Harnblase oder ein anderes Hohlorgan oder ein anderer natürlicher Hohlraum im Körper 10 eines Patienten. Alternativ ist der Hohlraum 12 für eine diagnostische, therapeutische und/oder chirurgische Maßnahme künstlich erzeugt.

Das medizinische Instrument 20 umfasst einen proximalen Bereich 22, der für eine Anordnung außerhalb des Körpers 10 eines Patienten vorgesehen und ausgebildet ist. In dem proximalen Bereich 22 des Instruments 20 sind unter anderem Einrichtungen zur Handhabung und Steuerung des medizinischen Instruments 20 vorgesehen. Dazu kann ein Arbeitselement zählen, das in Figur 1 nicht dargestellt ist. Ein proximaler Bereich 28 des medizinischen Instruments 20 ist zur Anordnung im Körper 10 eines Patienten und insbesondere in dem Hohlraum 12 in dem Körper 10 vorgesehen und ausgebildet. Das medizinische Instrument 20 umfasst einen Schaft 26, der den distalen Bereich 28 bildet und bis zu dem proximalen Bereich 22 reicht.

Das medizinische Instrument 20 kann ein Endoskop 30 mit einem proximalen Ende 32, das beispielsweise durch ein Okular gebildet wird und aus dem proximalen Bereich 22 des medizinischen Instruments 20 herausragt, umfassen. Ein Schaft 36 des Endoskops 30 ist in dem Schaft 26 des medizinischen Instruments 20 angeordnet. Ein distales Ende 38 des Endoskops 30 ist in dem distalen Bereich 28 des medizinischen Instruments 20, insbesondere nahe dessen äußerst distalem Ende angeordnet.

Das Endoskop 30 kann Teil des medizinischen Instruments 20 sein. Das Endoskop 30 kann mit dem medizinischen Instrument starr und nicht zerstörungsfrei lösbar verbunden sein. Alternativ kann das Endoskop 30 mit dem medizinischen Instrument zerstörungsfrei lösbar verbunden sein. Alternativ kann das medizinische Instrument 20 für ein Endoskop 30 ausgebildet sein, ohne dass das Endoskop 30 notwendiger oder dauernder Bestandteil des medizinischen Instruments 20 ist.

Das medizinische Instrument 20 weist eine erste Kupplungseinrichtung 42 in dem proximalen Bereich 22 auf. Die erste Kupplungseinrichtung 42 umfasst beispielsweise eine Luer-Kupplung oder eine Luer-Lock-Kupplung und/oder eine Schlauchtülle. Mittels der Kupplungseinrichtung 42 ist das medizinische Instrument 20 über einen Schlauch oder eine andere Fluidleitung mit einem Fluidreservoir zur Bereitstellung eines Spülfluids verbindbar.

Das medizinische Instrument 20 umfasst ferner einen ersten Fluidkanal 46 in dem Schaft 26 des medizinischen Instruments. Bei dem dargestellten Beispiel ist der Schaft 36 des Endoskops 30 so innerhalb des Schafts 26 des medizinischen Instruments 20 angeordnet, dass der erste Fluidkanal 46 den Schaft 36 des Endoskops 30 mantelförmig umgibt. Anders ausgedrückt weist der Querschnitt des ersten Fluidkanals 46 eine ringförmige Topologie auf und umschließt den Querschnitt des Schafts 36 des Endoskops 30.

Eine Öffnung 48 in dem distalen Bereich 28 des medizinischen Instruments 20 bildet das distale Ende des ersten Fluidkanals 46 und einen Auslass, durch den Fluid aus dem ersten Fluidkanal 46 aus und in den Hohlraum 12 im Körper 10 eintreten kann.

Zwischen der ersten Kupplungseinrichtung 42 und dem ersten Fluidkanal 46 ist ein Strömungswiderstandsmittel 44 mit einem reduzierten Querschnitt 45 angeordnet.

Die erste Kupplungseinrichtung 42, das erste Strömungswiderstandsmittel 44, der erste Fluidkanal 46 und die erste Öffnung 48 bilden eine Zuleitungseinrichtung zum Zuführen eines Fluids, insbesondere einer Spülflüssigkeit oder eines anderen Spülfluids, zu dem Hohlraum 12. Die geringste Querschnittsfläche der Zuleitungseinrichtung 42, 44, 46, 48 liegt bei dem reduzierten Querschnitt 45 des ersten Strömungswiderstandsmittels 44 vor. Der Strömungswiderstand des Strömungswiderstandsmittels 44 bestimmt den Strömungswiderstand der gesamten Zuleitungseinrichtung maßgeblich oder im Wesentlichen. Insbesondere beträgt der Strömungswiderstand des Strömungswiderstandsmittels 44 mindestens die Hälfte oder mindestens zwei Drittel oder mindestens drei Viertel oder mindestens vier Fünftel oder mindestens neun Zehntel des Strömungswiderstands der gesamten Zuleitungseinrichtung 42, 44, 46, 48.

Das medizinische Instrument 20 umfasst ferner eine zweite Kupplungseinrichtung 52 in dem proximalen Bereich 22. Die zweite Kupplungseinrichtung 22 umfasst insbesondere eine Luer-Kupplung oder eine Luer-Lock-Kupplung und/oder eine Schlauchtülle. Mittels der zweiten Kupplungseinrichtung 52 ist das medizinische Instrument 20 durch einen Schlauch oder eine andere Fluidleitung mit einem Behälter zum Aufnehmen von gebrauchtem Spülfluid verbindbar.

In dem Schaft 26 des medizinischen Instruments 20 ist ferner eine zweite Fluidleitung 56 vorgesehen. Eine zweite Öffnung 58 in dem distalen Bereich 28 des medizinischen Instruments 20 bildet das distale Ende des zweiten Fluidkanals 56 und damit einen Einlass.

Zwischen dem proximalen Ende des zweiten Fluidkanals 56 und der zweiten Kupplungseinrichtung 52 ist ein zweites Strömungswiderstandsmittel 54 mit einem reduzierten Querschnitt 55 vorgesehen.

Die zweite Öffnung 58 in dem distalen Bereich 28 des medizinischen Instruments 20, der zweite Fluidkanal 56, das zweite Strömungswiderstandsmittel 54 und die zweite Kupplungseinrichtung 52 bilden eine Ableitungseinrichtung zum Ableiten eines Fluids aus dem Hohlraum 12 in dem Körper 10. Die minimale Querschnittsfläche der Ableitungseinrichtung 52, 54, 56, 58 liegt bei dem reduzierten Querschnitt 55 des zweiten Strömungswiderstandsmittels 54 vor. Der Strömungswiderstand der Ableitungseinrichtung 52, 54, 56, 58 wird deshalb maßgeblich oder im Wesentlichen durch das zweite Strömungswiderstandsmittel 54 bestimmt. Insbesondere beträgt der Strömungswiderstand des zweiten Strömungswiderstandsmittels 54 mindestens ein Zehntel oder mindestens ein Fünftel oder mindestens ein Viertel oder mindestens ein Drittel oder mindestens die Hälfte oder mindestens zwei Drittel oder mindestens drei Viertel oder mindestens vier Fünftel oder mindestens neun Zehntel des Strömungswiderstands der gesamten Ableitungseinrichtung 52, 54, 56,58.

Bei dem dargestellten Beispiel ist die Querschnittsfläche im Bereich des reduzierten Querschnitts 45 des ersten Strömungswiderstandsmittels 44 ist kleiner als die Querschnittsfläche im Bereich des reduzierten Querschnitts 55 des zweiten Strömungswiderstandsmittels 54. Dadurch ist insbesondere der Strömungswiderstand der Zuleitungseinrichtung 42, 44, 46, 48 größer als der Strömungswiderstand der Ableitungseinrichtung 52, 54, 56, 58.

Bei dem dargestellten medizinischen Instrument 20 kann die Ableitungseinrichtung 52, 54, 56, 58 nicht verschlossen werden. Anders ausgedrückt gibt es keine vorgesehene Konfiguration bzw. keinen vorgesehenen Betriebszustand des medizinischen Instruments 20, bei dem die Ableitungseinrichtung 52, 54, 56, 58 verschlossen wäre.

Bei der vorgesehenen Verwendung des medizinischen Instruments 20 wird die erste Kupplungseinrichtung 42 mittels eines Schlauchs oder einer anderen Fluidleitung mit einem Beutel oder einem anderen Reservoir für ein frisches, steriles Spülfluid, insbesondere eine Spülflüssigkeit, verbunden. Dieses Reservoir wird über dem Hohlraum 12 angeordnet. Die Höhendifferenz erzeugt eine Druckdifferenz. Die Druckdifferenz bewirkt ein in Figur 1 durch Pfeile angedeutetes Fließen von Fluid in die erste Kupplungseinrichtung 42, durch das erste Strömungswiderstandsmittel 44, den ersten Fluidkanal 46 und die erste Öffnung 48 in den Hohlraum 12 hinein. Der dadurch entstehende Druck in dem Hohlraum 12 - genauer: die Druckdifferenz zwischen dem Hohlraum 12 und der Umgebung - bewirkt ein Abfließen von Fluid aus dem Hohlraum 12 durch die zweite Öffnung 58, den zweiten Fluidkanal 56, das zweite Strömungswiderstandsmittel 54 und die zweite Kupplungseinrichtung 52. Die zweite Kupplungseinrichtung 52 ist insbesondere durch einen Schlauch oder eine andere Fluidleitung mit einem Auffanggefäß für das Fluid verbunden.

Wenn, wie in Figur 1 angedeutet, der Strömungswiderstand der Ableitungseinrichtung 52, 54, 56, 58 geringer ist als der Strömungswiderstand der Zuleitungseinrichtung 42, 44, 46, 48, stellt sich in dem Hohlraum 12 ein Druck ein, der näher bei dem an der zweiten Kupplungseinrichtung 52 vorliegenden Druck als bei dem an der ersten Kupplungseinrichtung 42 vorliegenden Druck liegt. Dieser im Vergleich zu vielen möglichen Konfigurationen herkömmlicher medizinischer Instrumente niedrige Druck in dem Hohlraum 12 schont den Patienten.

Wenn, wie in Figur 1 angedeutet, die Ableitungseinrichtung 52, 54, 56, 58 nicht verschließbar ist, kann der in dem Hohlraum 12 sich einstellende Druck nicht - wie bei möglichen Konfigurationen vieler herkömmlicher medizinischer Instrumente - den an der ersten Kupplungseinrichtung 42 vorliegenden Druck erreichen. Dadurch wird der Patient geschont.

Figur 2 zeigt eine schematische Darstellung eines Schnitts durch einen Körper 10 eines Patienten und durch ein weiteres medizinisches Instrument 20, das in einigen Merkmalen, Eigenschaften und Funktionen dem anhand der Figur 1 dargestellten medizinischen Instrument ähnelt. Die Schnittebene und die Art der Darstellung in Figur 2 entsprechen denjenigen der Figur 1. Nachfolgend sind insbesondere Eigenschaften, Merkmale und Funktionen des in Figur 2 gezeigten medizinischen Instruments 20 beschrieben, in denen dieses sich von dem anhand der Figur 1 dargestellten medizinischen Instrument unterscheidet.

Bei dem in Figur 2 gezeigten medizinischen Instrument sind die Strömungswiderstandsmittel 44, 54 als Ventile, insbesondere als Kükenventile ausgebildet. Bei dem dargestellten Beispiel ist der Strömungswiderstand des ersten Strömungswiderstandsmittel 44 größer als der Strömungswiderstands des zweiten Strömungswiderstandsmittels 54. Das zweite Strömungswiderstandsmittel 54 weist insbesondere mehrere verschiedene Konfigurationen mit unterschiedlichen Strömungswiderständen auf. Das zweite Strömungswiderstandsmittel 54 ist jedoch insbesondere so ausgebildet, dass es nicht vollständig verschlossen werden kann. Anders ausgedrückt kann in jeder für den bestimmungsgemäßen Gebrauch des medizinischen Instruments 20 vorgesehenen Konfiguration des zweiten Strömungswiderstandsmittels 54 Fluid durch das zweite Strömungswiderstandsmittel 54 fließen.

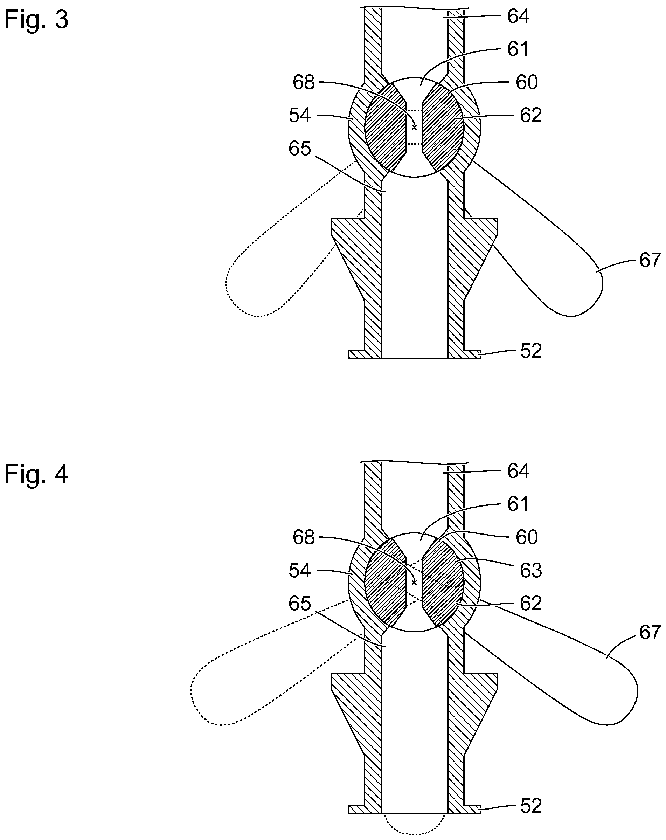

Figur 3 zeigt eine vergrößerte schematische Darstellung eines Schnitts durch das als Kükenventil ausgebildete zweite Strömungswiderstandsmittel 54 des anhand der Figur 2 dargestellten medizinischen Instruments. Ferner ist in Figur 3 die an das Kükenventil 54 angrenzende Kupplungseinrichtung 52 angedeutet. Die Schnittebene der Figur 3 entspricht der Schnittebene der Figur 2.

Das Kükenventil 54 umfasst ein als Küken 60 bezeichnetes konisches Bauteil. Das Küken 60 ist um seine orthogonal zur Schnittebene der Figur 3 angeordnete Symmetrieachse- und Rotationsachse 68 rotierbar. Das Küken 60 weist zwei Durchgangsbohrungen 61, 62 mit unterschiedlichen Querschnitten auf. Die Schnittebene der Figur 3 liegt innerhalb der ersten Durchgangsbohrung 61 und außerhalb der zweiten Durchgangsbohrung 62. Die zweite Durchgangsbohrung 62 ist deshalb nur durch gestrichelte Konturen angedeutet. Die Durchgangsbohrungen 61, 62 sind jeweils orthogonal zu der Symmetrie- und Rotationsachse 68 des Kükens 60 angeordnet. Die Enden der Durchgangsbohrungen 61, 62 können, wie in Figur 3 angedeutet, konisch ausgebildet sein, um Verwirbelungen eines die Durchgangsbohrungen 61, 62 durchströmenden Fluids zu mindern oder zu verhindern.

Das Küken 60 ist zwischen einem ersten Fluidkanal 64 und einem zweiten Fluidkanal 65 in einer konischen Bohrung mit korrespondierender Gestalt angeordnet. Bei der in Figur 3 gezeigten Position des Kükens 60 verbindet die erste Durchgangsbohrung 61 die Fluidkanäle 64, 65 des Kükenventils 54. Bei einer gegenüber der in Figur 3 angedeuteten Position um 90 Grad rotierten Position des Kükens 60 verbindet die zweite Durchgangsbohrung 62 des Kükens 60 die Fluidkanäle 64, 65 des Kükenventils 54.

Ein manuell betätigbarer Hebel 67 ist hinter der Schnittebene der Figur 3 angeordnet. Der Hebel 67 ist starr mit dem Küken 60 verbunden und mit diesem zusammen um die Symmetrie- und Rotationsachse 68 rotierbar.

Der Hebel 67 ist in Figur 3 zweimal in verschiedenen Positionen dargestellt, einmal mit einer durchgezogenen und einmal mit einer gestrichelten Linie. Der Hebel 67 nimmt beispielsweise die in durchgezogener Linie dargestellte Position ein, wenn das Küken 60 die in Figur 3 gezeigte Position einnimmt, bei der die erste Durchgangsbohrung 61 die Fluidkanäle 64, 65 des Kükenventils 54 verbindet. Wenn der Hebel 67 die in Figur 3 in gestrichelter Linie angedeutete Position annimmt, ist das Küken 60 gegenüber der in Figur 3 gezeigten Position um 90 Grad rotiert, so dass die zweite Durchgangsbohrung 62 die Fluidkanäle 64, 65 des Kükenventils 54 miteinander verbindet. Da die Durchgangsbohrungen 61, 62 des Kükens 60 unterschiedliche Durchmesser und Querschnitte und damit unterschiedliche Strömungswiderstände aufweisen, weist das gesamte Kükenventil 54 in beiden Positionen unterschiedliche Strömungswiderstände auf.

Die Öffnungen der Durchgangsbohrungen 61, 62 können so ausgebildet und angeordnet sein, dass auch während der Rotation des Kükens 60 um die Rotationsachse 68 zwischen den beiden vorgesehenen Positionen zu keinem Zeitpunkt die Verbindung zwischen den Fluidkanälen 64, 65 des Kükenventils 54 vollständig verschlossen ist.

Das erste Strömungswiderstandsmittel des anhand der Figur 2 dargestellten medizinischen Instruments 20 kann baugleich mit oder ähnlich dem anhand der Figur 3 dargestellten Kükenventil 54 sein.

Figur 4 zeigt eine schematische Darstellung eines weiteren Kükenventils 54, das in einigen Merkmalen, Eigenschaften und Funktionen dem anhand der Figur 3 dargestellten Kükenventil ähnelt. Die Art der Darstellung, insbesondere die Schnittebene der Figur 4 entspricht derjenigen der Figur 3. Nachfolgend sind insbesondere Merkmale, Eigenschaften und Funktionen des Kükenventils 54 beschrieben, in denen dieses sich von dem anhand der Figur 3 dargestellten Kükenventil unterscheidet.

Das in Figur 4 gezeigte Kükenventil 54 kann Teil des anhand der Figur 2 dargestellten medizinischen Instruments sein, und zwar sowohl als Teil der Zuleitungseinrichtung als auch als Teil der Ableitungseinrichtung.

Das in Figur 4 gezeigte Kükenventil 54 unterscheidet sich von dem anhand der Figur 3 dargestellten Kükenventil insbesondere dadurch, dass das Küken 60 drei Durchgangsbohrungen 61, 62, 63 mit unterschiedlichen Durchmessern und Querschnitten aufweist. Die Durchgangsbohrungen 61, 62 des Kükens 60 sind in unterschiedlichen Ebenen orthogonal zu der Rotationsachse 68 des Kükens 60 angeordnet. Der Schnittebene der Figur 4 schneidet die erste Durchgangsbohrung 61, nicht jedoch die zweite Durchgangsbohrung 62 und die dritte Durchgangsbohrung 63. Deshalb sind die Konturen der zweiten Durchgangsbohrung 62 und der dritten Durchgangsbohrung 63 lediglich durch gestrichelte Linien angedeutet.

Die Durchgangsbohrungen 61, 62, 63 verlaufen in unterschiedlichen Richtungen. Insbesondere sind die Längsachsen der Durchgangsbohrungen 61, 62, 63 jeweils um 60 Grad voneinander beabstandet.

In Figur 4 sind drei verschiedene Positionen des mit dem Küken 60 starr verbundenen Hebels 67 angedeutet. In der mit einer durchgezogenen Linie gezeigten Position des Hebels 67 nimmt das Küken 60 die in Figur 4 dargestellte Position ein. Die erste Durchgangsbohrung 61 verbindet die Fluidkanäle 64, 65 des Kükenventils 54. In den anderen beiden, durch gestrichelte Linien angedeuteten Positionen des Hebels 67 (eine davon weitgehend durch die Kupplungseinrichtung 42, 52 verdeckt) ist das Küken 60 gegenüber der in Figur 4 gezeigten Position um 60 Grad bzw. um 120 Grad rotiert. Entsprechend verbindet nicht die erste Durchgangsbohrung 61, sondern die zweite Durchgangsbohrung 62 bzw. die dritte Durchgangsbohrung 63 die Fluidkanäle 64, 65 des Kükenventils 54.

Da die Durchgangskanäle 61, 62, 63 unterschiedliche Durchmesser und Querschnitte und damit unterschiedliche Strömungswiderstände aufweisen, weist das gesamte Kükenventil 54 in den drei Konfigurationen unterschiedliche Strömungswiderstände auf.

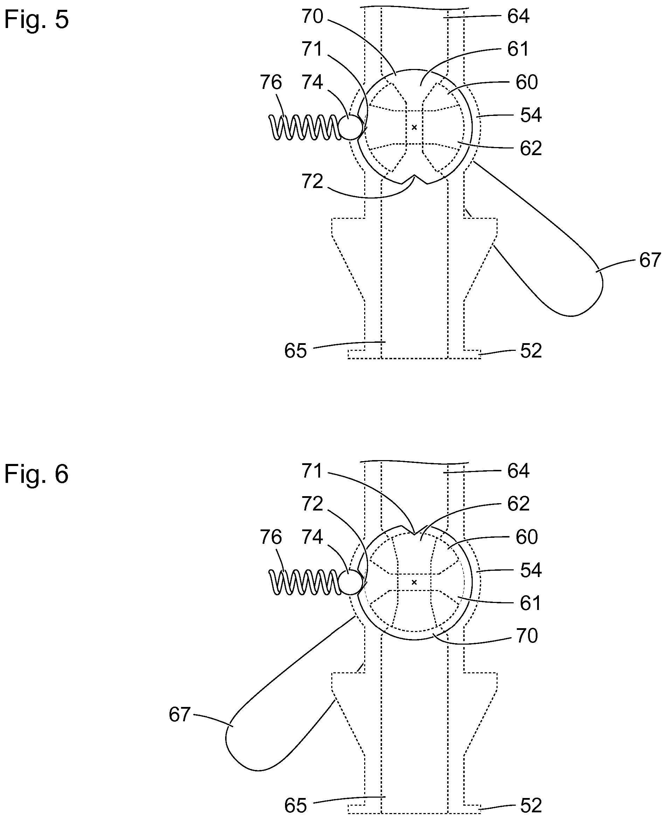

Figur 5 zeigt eine schematische Darstellung einer Variante des anhand der Figur 3 dargestellten Kükenventils 54. Die Zeichenebene der Figur 5 ist parallel zu der Schnittebene der Figur 3. Die bereits anhand der Figur 3 dargestellten Merkmale sind weitgehend lediglich durch gestrichelte Konturen angedeutet.

Die in Figur 5 gezeigte Variante des Kükenventils 54 weist eine mit dem Küken 60 starr verbundene und im Wesentlichen kreisförmige Scheibe 70 auf. Die Scheibe 70 weist an ihrem äußeren Umfang zwei Nuten 71, 72 auf. Ferner umfasst das Kükenventil 54 eine Kugel 74 und eine Feder 76, die in einer in Figur 5 nicht dargestellten Sackbohrung in einem in Figur 5 nicht dargestellten Gehäuseabschnitt des Kükenventils 54 angeordnet sind. Die Schraubenfeder 76 drückt die Kugel 74 gegen den äußeren Umfang der Scheibe 70.

Bei der in Figur 5 gezeigten Konfiguration liegt die Kugel 74 in die erste Nut 71 am äußeren Umfang der Scheibe 70. Der Eingriff der Kugel 74 in die erste Nut 71 hält die Scheibe 70 und damit auch das Küken 60 und den Hebel 67 in den in Figur 5 angedeuteten Positionen. Eine Rotation des Hebels 67, des Kükens 60 und der Scheibe 70 von der in Figur 5 gezeigten Position weg setzt eine Auslenkung der Kugel 74 aus der in Figur 5 gezeigten Position und eine Kompression der Schraubenfeder 76 voraus.

Figur 6 zeigt eine weitere schematische Darstellung des Kükenventils 54 aus Figur 5. Bei der in Figur 6 gezeigten Konfiguration und den in Figur 6 gezeigten Positionen des Hebels 67, des Kükens 60 und der Scheibe 70 greift die Kugel 74 in die zweite Nut 72 an dem äu-ßeren Umfang der Scheibe 70 ein. Die zweite Durchgangsbohrung 62 verbindet die Fluidkanäle 64, 65 des Kükenventils 54.

Die Nuten 71, 72 an dem äußeren Umfang der Scheibe 70, die Kugel 74 und die Schraubenfeder 76 wirken somit als Rasteinrichtung. Das Einrasten bei den in den Figuren 5 und 6 angedeuteten Positionen ist für Anwender spürbar und vereinfacht die Einstellung der beiden alternativen vorbestimmten Konfigurationen.

Abweichend von der Darstellung in den Figuren 5 und 6 können die Nuten 72 an dem äu-ßeren Umfang der Scheibe 70 so (insbesondere so tief) ausgebildet sein, dass ihre Ränder aneinander angrenzen, also kein kreisbogenförmiger Bereich der Oberfläche der Scheibe 70 vorliegt. In diesem Fall kann die durch die Schraubenfeder 76 erzeugte Kraft auf die Kugel 74 bei jeder Position zwischen den in den Figuren 5 und 6 angedeuteten Positionen eine Bewegung der Scheibe 70 und damit auch des Kükens 60 und des Hebels 67 hin zu einer der beiden in den Figuren 5 und 6 dargestellten vorbestimmten Positionen bewirken.

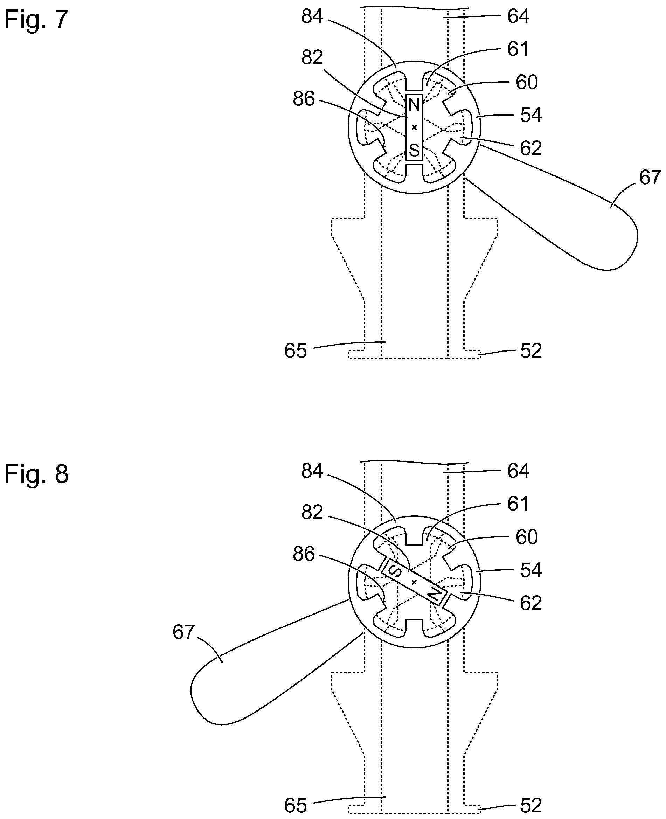

Figur 7 zeigt eine schematische Darstellung einer Variante des anhand der Figur 4 dargestellten Kükenventils 54. Die Art der Darstellung in Figur 7 entspricht derjenigen der Figuren 5 und 6.

Bei der in Figur 7 dargestellten Variante des Kükenventils 54 ist das Küken 60 mit einem Magneten 82 mechanisch starr verbunden. Um den Magneten 82 herum ist ein Magnetflussleiter 84 angeordnet. Der Magnetflussleiter 84 ist beispielsweise aus einem weichmagnetischen eisenhaltigen Material gebildet. Der Magnetflussleiter 84 weist eine im Wesentlichen kreisförmige oder polygonale Grundstruktur auf. Abweichend von einer idealen Kreisform weist der Magnetflussleiter 84 nach radial innen zu dem Magneten 82 und der Rotationsachse 68 hin gerichtete Polflächen 86 auf. Die Polflächen 86 sind paarweise einander gegenüber angeordnet, so dass in mehreren vorbestimmten Positionen des Magneten 82 und damit auch des Kükens 60 und des Hebels 67, die mit dem Magneten 82 starr verbunden sind, beide Pole des Magneten 82 jeweils einer Polfläche 86 des Magnetflussleiters 84 gegenüberliegen. Diese Konfigurationen sind energetisch bevorzugt. Der Magnet 82 und der Magnetflussleiter 84 mit den Polflächen 86 bilden somit eine Rasteinrichtung, die mehrere vorbestimmte Positionen des Magneten 82, des Kükens 60 und des Hebels 67 definiert.

Figur 8 zeigt eine weitere schematische Darstellung des Kükenventils 54 aus Figur 7. Die Art der Darstellung entspricht derjenigen der Figur 7. In Figur 8 ist das Kükenventil 54 in einer weiteren Konfiguration gezeigt, die aus der in Figur 7 gezeigten Konfiguration durch Rotation des Hebels 67, des Kükens 60 und des Magneten 82 um die Rotationsachse 68 hervorgeht. Der Magnet 82, das Küken 60 und der Hebel 67 werden in beiden in den Figuren 7 und 8 gezeigten Konfigurationen bzw. Positionen magnetisch gehalten. Ein magnetisches Rastmoment muss überwunden werden, um den Hebel 67 und mit ihm das Küken 60 und den Magneten 82 von einer der energetisch bevorzugten Positionen in eine andere zu bewegen.

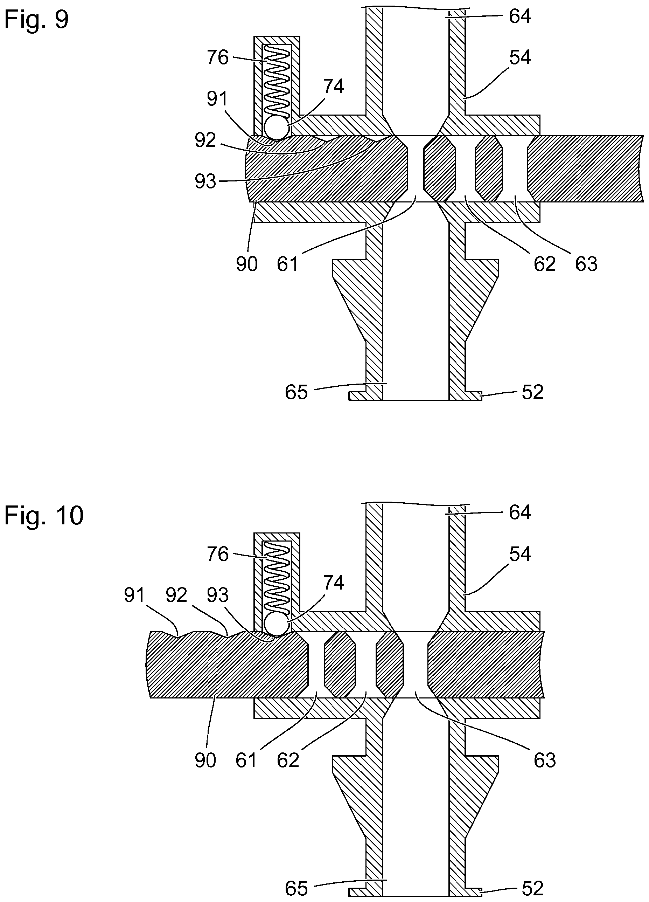

Figur 9 zeigt eine schematische Darstellung eines Schnitts durch ein weiteres Strömungswiderstandsmittel 54, das Bestandteil des anhand der Figur 2 dargestellten medizinischen Instruments in dessen Zuleitungseinrichtung oder in dessen Ableitungseinrichtung sein kann. Die Art der Darstellung entspricht denjenigen der Figuren 3 und 4. Nachfolgend sind insbesondere Merkmale, Eigenschaften und Funktionen, in denen das in Figur 9 gezeigte Strömungswiderstandsmittel 54 sich von den anhand der Figuren 3 bis 8 dargestellten Strömungswiderstandsmitteln unterscheidet, beschrieben.

Das in Figur 9 gezeigte Strömungswiderstandsmittel 54 weist anstelle eines Kükens einen Schieber 90 auf. Der Schieber 90 ist in einem korrespondierenden Kanal in dem Strömungswiderstandsmittel 54 so spiel- und reibungsarm geführt, dass der Schieber 90 entlang eines geraden Pfads bewegt werden kann. Der Schieber 90 weist mehrere Durchgangsbohrungen 61, 62, 63 mit unterschiedlichen Querschnitten und entsprechend unterschiedlichen Strömungswiderständen auf.

Ferner weist der Schieber 90 mehrere Nuten 92 auf. In einer zu dem Schieber 90 hin offenen Sackbohrung in dem Gehäuse des Strömungswiderstandsmittels 54 sind eine Rastkugel 74 und eine Schraubenfeder 76 angeordnet. In vorbestimmten Positionen des Schiebers 90 greift die Rastkugel 74 in jeweils eine der Nuten 92 ein. Gleichzeitig verbindet jeweils eine der Durchgangsbohrungen 61, 62, 63 - bei der in Figur 9 gezeigten Konfiguration die erste Durchgangsbohrung 61 - zwei Fluidkanäle 64, 65 des Strömungswiderstandsmittels 54. Die Konfiguration des Strömungswiderstandsmittels 54, nämlich die Position des Schiebers 90 kann manuell zwischen den durch den Eingriff der Kugel 74 in die Nuten 91, 92, 93 definierten Positionen bewegt werden.

Figur 10 zeigt eine weitere schematische Darstellung des Strömungswiderstandsmittels 54 aus Figur 9. Die Art der Darstellung entspricht derjenigen der Figur 9.

In Figur 10 ist das Strömungswiderstandsmittel 54 in einer anderen Konfiguration dargestellt, bei der eine andere Durchgangsbohrung 63 in dem Schieber 90 die Fluidkanäle 64, 65 des Strömungswiderstandsmittels 54 verbindet.

### Bezugszeichen

- 10: Körper eines Patienten
- 12: Hohlraum im Körper 10
- 20: Medizinisches Instrument
- 22: proximaler Bereich des medizinischen Instruments 20
- 26: Schaft des medizinischen Instruments 20
- 28: distaler Bereich des medizinischen Instruments 20
- 30: Endoskop
- 32: proximales Ende des Endoskops 30
- 36: Schaft des Endoskops 30
- 38: distales Ende des Endoskops 30
- 42: erste Kupplungseinrichtung in dem proximalen Bereich 22
- 44: erstes Strömungswiderstandsmittel in dem proximalen Bereich 22, insb. Ventil
- 45: reduzierter Querschnitt in dem ersten Strömungswiderstandsmittel
- 46: erster Fluidkanal in dem Schaft 26
- 48: erste Öffnung als Auslass des ersten Fluidkanals 46 in dem distalen Bereich 28
- 52: zweite Kopplungseinrichtung in dem proximalen Bereich 22
- 54: zweites Strömungswiderstandsmittel in dem proximalen Bereich 22, insb. Ventil
- 55: reduzierter Querschnitt in dem zweiten Strömungswiderstandsmittel
- 56: zweiter Fluidkanal in dem Schaft 26
- 58: zweite Öffnung als Einlass des zweiten Fluidkanals 56 in dem distalen Bereich 28
- 60: Küken des Ventils 54
- 61: erste Durchgangsbohrung in dem Küken 62 Rasteinrichtung
- 62: zweite Durchgangsbohrung in dem Küken 62 Rasteinrichtung
- 63: dritte Durchgangsbohrung in dem Küken 62 Rasteinrichtung
- 64: erster Fluidkanal des Ventils 54
- 65: zweiter Fluidkanal des Ventils 54
- 67: Hebel des Ventils 54
- 68: Rotationsachse des Kükens 60 und des Hebels 67
- 70: mit dem Küken 60 verbundene, im Wesentlichen kreisförmige Scheibe
- 71: erste Rastnut am Umfang der Scheibe 70
- 72: zweite Rastnut am Umfang der Scheibe 70
- 74: Rastkugel
- 76: Schraubenfeder
- 82: Magnet
- 84: Magnetflussleiter
- 86: Polfläche des Magnetflussleiters 84
- 90: Schieber
- 91: erste Rastnut an der äußeren Oberfläche des Schiebers 90
- 92: zweite Rastnut an der äußeren Oberfläche des Schiebers 90
- 93: dritte Rastnut an der äußeren Oberfläche des Schiebers 90

## Patentansprüche

1. **Medizinisches Instrument** (20) für diagnostische, therapeutische oder chirurgische Maßnahmen in einem Hohlraum (12) im Körper (10) eines Patienten, mit:
einem **proximalen Bereich** (22), der zur Anordnung außerhalb des Körpers (10) vorgesehen und ausgebildet ist;
einem **distalen Bereich** (28), der zur Anordnung innerhalb eines Hohlraums (12) in dem Körper (10) vorgesehen und ausgebildet ist;
einer **Zuleitungseinrichtung** (42, 44, 46, 48) zum Zuführen eines Fluids zu dem Hohlraum (12), wobei die Zuleitungseinrichtung (42, 44, 46, 48) von einer ersten Kupplung (42) in dem proximalen Bereich (22) des medizinischen Instruments (20) bis zu einem Auslass (48) in dem distalen Bereich (28) reicht;
einer **Ableitungseinrichtung** (52, 54, 56, 58) zum Ableiten eines Fluids aus dem Hohlraum (12), wobei die Ableitungseinrichtung (52, 54, 56, 58) von einem Einlass (58) in dem distalen Bereich (28) des medizinischen Instruments (20) bis zu einer zweiten Kupplung (52) in dem proximalen Bereich (22) des medizinischen Instruments (20) reicht;
wobei der **Strömungswiderstand der Zuleitungseinrichtung** (42, 44, 46, 48) **größer** ist als der Strömungswiderstand der Ableitungseinrichtung (52, 54, 56, 58),
wobei zumindest entweder
die Zuleitungseinrichtung (42, 44, 46, 48) ein **Strömungswiderstandsmittel** (44), das den **Strömungswiderstand** der Zuleitungseinrichtung (42, 44, 46, 48) im Wesentlichen bestimmt, aufweist oder
die Ableitungseinrichtung (52, 54, 56, 58) ein **Strömungswiderstandsmittel** (54), das den **Strömungswiderstand** der Ableitungseinrichtung (52, 54, 56, 58) im Wesentlichen bestimmt, aufweist,
**dadurch gekennzeichnet dass**, das Strömungswiderstandsmittel (44; 54) eine **endliche Anzahl** alternativer vorbestimmter **Konfigurationen** mit unterschiedlichen Strömungswiderständen aufweist, und das Strömungswiderstandsmittel (44; 54) vorgesehen und ausgebildet ist, um ausschließlich in den vorbestimmten Konfigurationen betrieben zu werden.

2. Medizinisches Instrument (20) nach dem vorangehenden Anspruch, bei dem die **Ableitungseinrichtung** (52, 54, 56, 58) bei der vorgesehenen Verwendung des medizinischen Instruments (20) **nicht verschließbar** oder nur auf eine Weise verschließbar ist, die bei Überschreiten eines vorbestimmten Drucks ein Öffnen der Ableitungseinrichtung (52, 54, 56, 58) zur Folge hat.

3. Medizinisches Instrument (20) nach einem der vorangehenden Ansprüche, bei dem die Ableitungseinrichtung (52, 54, 56, 58) ein Sicherheitsventil, das bei Überschreiten eines vorbestimmten Drucks in der Ableitungseinrichtung öffnet, umfasst.

4. Medizinisches Instrument (20) nach einem der vorangehenden Ansprüche, wobei bei dem Strömungswiderstandsmittel (44; 54) die **geringste Querschnittsfläche** (45; 55) der Zuleitungseinrichtung (42, 44, 46, 48) oder der Ableitungseinrichtung (52, 54, 56, 58) vorliegt.

5. Medizinisches Instrument (20) nach Anspruch 1 und 3, bei dem das Strömungswiderstandsmittel (44; 54) einen **einstellbaren Strömungswiderstand** aufweist.

6. Medizinisches Instrument (20) nach einem der vorangehenden Ansprüche, bei dem das Strömungswiderstandsmittel (44; 54) eine **Rasteinrichtung** (72, 74, 76; 82, 84; 92), die nur die vorbestimmten Konfigurationen des Strömungswiderstandsmittels (44; 54) zulässt oder die vorbestimmten Konfigurationen gegenüber anderen Konfigurationen bevorzugt, aufweist.

7. Medizinisches Instrument (20) nach einem der Ansprüche 5 und 6, bei dem
das Strömungswiderstandsmittel (44; 54) ein **rotierbares** oder entlang eines geraden oder gekrümmten Pfads **bewegbares Bauelement** (60; 90) mit mehreren Durchgangsbohrungen (61, 62, 63) aufweist,
in jeder vorbestimmten Konfiguration des Strömungswiderstandsmittels (44; 54) Fluid durch eine oder mehrere der Durchgangsbohrungen (61, 62, 63) strömen kann.

8. Medizinisches Instrument (20) nach dem vorangehenden Anspruch, bei dem
die Durchgangsbohrungen (61, 62, 63) **unterschiedliche Querschnitte** aufweisen,
in jeder vorbestimmten Konfiguration des Strömungswiderstandsmittels (44, 54) Fluid durch eine der Durchgangsbohrungen (61, 62, 63) strömen kann.

9. Medizinisches Instrument (20) nach einem der vorangehenden Ansprüche, bei dem
die Ableitungseinrichtung (52, 54, 56, 58) ein **Strömungswiderstandsmittel** (54) aufweist,
das Strömungswiderstandsmittel (54) der Ableitungseinrichtung (52, 54, 56, 58) **in keiner** möglichen oder in keiner vorgesehenen Konfiguration des medizinischen Instruments (20) die Ableitungseinrichtung (52, 54, 56, 58) **vollständig verschließt.**

10. Medizinisches Instrument (20) nach einem der vorangehenden Ansprüche, bei dem
sowohl die Zuleitungseinrichtung (42, 44, 46, 48) als auch die Ableitungseinrichtung (52, 54, 56, 58) jeweils ein **Strömungswiderstandsmittel** (44, 54) aufweist,
der **Strömungswiderstand** des Strömungswiderstandsmittels (44) der Zuleitungseinrichtung (42, 44, 46, 48) **größer** ist als der Strömungswiderstand des Strömungswiderstandsmittels (54) der Ableitungseinrichtung (52, 54, 56, 58).

11. Medizinisches Instrument (20) nach dem vorangehenden Anspruch, bei dem der **minimale Querschnitt** (45) des Strömungswiderstandsmittels (44) der Zuleitungseinrichtung (42, 44, 46, 48) **kleiner** ist als der minimale Querschnitt (55) des Strömungswiderstandsmittels (54) der Ableitungseinrichtung (52, 54, 56, 58).

## Claims

1. Medical instrument (20) for diagnostic, therapeutic or surgical measures in a cavity (12) in the body (10) of a patient, comprising:
a proximal region (22), which is provided and designed for arrangement outside the body (10);
a distal region (28), which is provided and designed for arrangement inside a cavity (12) in the body (10) ;
a supply structure (42, 44, 46, 48) for delivering a fluid to the cavity (12), wherein the supply structure (42, 44, 46, 48) reaches from a first coupling (42) in the proximal region (22) of the medical instrument (20) to an outlet (48) in the distal region (28);
a discharge structure (52, 54, 56, 58) for discharging a fluid from the cavity (12), wherein the discharge structure (52, 54, 56, 58) reaches from an inlet (58) in the distal region (28) of the medical instrument (20) to a second coupling (52) in the proximal region (22) of the medical instrument (20);
wherein the flow resistance of the supply structure (42, 44, 46, 48) is greater than the flow resistance of the discharge structure (52, 54, 56, 58),
wherein at least either
the supply structure (42, 44, 46, 48) has a flow resistance means (44) which substantially determines the flow resistance of the supply structure (42, 44, 46, 48), or
the discharge structure (52, 54, 56, 58) has a flow resistance means (54) which substantially determines the flow resistance of the discharge structure (52, 54, 56, 58),
wherein
the flow resistance means (44; 54) has a finite number of alternative predetermined configurations with different flow resistances, and
the flow resistance means (44; 54) is provided and designed to be operated exclusively in the predetermined configurations.

2. Medical instrument (20) according to the preceding claim, in which the discharge structure (52, 54, 56, 58), during the intended use of the medical instrument (20), is not closable or is closable only in such a way that the discharge structure (52, 54, 56, 58) opens as a result of a predetermined pressure being exceeded.

3. Medical instrument (20) according to one of the preceding claims, in which the discharge structure (52, 54, 56, 58) comprises a safety valve which opens if a predetermined pressure in the discharge structure is exceeded.

4. Medical instrument (20) according to one of the preceding claims, wherein the smallest cross-sectional area (45; 55) of the supply structure (42, 44, 46, 48) or of the discharge structure (52, 54, 56, 58) is present in the flow resistance means (44; 54) .

5. Medical instrument (20) according to Claims 1 and 3, in which the flow resistance means (44; 54) has an adjustable flow resistance.

6. Medical instrument (20) according to one of the preceding claims, in which the flow resistance means (44; 54) has a latching structure (72, 74, 76; 82, 84; 92) which permits only the predetermined configurations of the flow resistance means (44; 54) or which prefers the predetermined configurations over other configurations.

7. Medical instrument (20) according to either of Claims 5 and 6, in which
the flow resistance means (44; 54) has a rotatable member (60; 90), or a member (60; 90) movable along a straight or curved path, with several through-bores (61, 62, 63),
fluid can flow through one or more of the through-bores (61, 62, 63) in each predetermined configuration of the flow resistance means (44; 54).

8. Medical instrument (20) according to the preceding claim, in which
the through-bores (61, 62, 63) have different cross sections,
fluid can flow through one of the through-bores (61, 62, 63) in each predetermined configuration of the flow resistance means (44, 54).

9. Medical instrument (20) according to one of the preceding claims, in which
the discharge structure (52, 54, 56, 58) has a flow resistance means (54),
the flow resistance means (54) of the discharge structure (52, 54, 56, 58) does not completely close the discharge structure (52, 54, 56, 58) in any possible configuration or any intended configuration of the medical instrument (20).

10. Medical instrument (20) according to one of the preceding claims, in which
the supply structure (42, 44, 46, 48) and the discharge structure (52, 54, 56, 58) each have a respective flow resistance means (44, 54),
the flow resistance of the flow resistance means (44) of the supply structure (42, 44, 46, 48) is greater than the flow resistance of the flow resistance means (54) of the discharge structure (52, 54, 56, 58).

11. Medical instrument (20) according to the preceding claim, in which the minimal cross section (45) of the flow resistance means (44) of the supply structure (42, 44, 46, 48) is smaller than the minimal cross section (55) of the flow resistance means (54) of the discharge structure (52, 54, 56, 58) .

## Revendications

1. Instrument médical (20) destiné à des interventions diagnostiques, thérapeutiques ou chirurgicales dans une cavité (12) dans le corps (10) d'un patient, comprenant :
une zone proximale (22) qui est prévue et réalisée pour être agencée à l'extérieur du corps (10) ;
une zone distale (28) qui est prévue et réalisée pour être agencée à l'intérieur d'une cavité (12) dans le corps (10) ;
un équipement d'amenée (42, 44, 46, 48) destiné à amener un fluide à la cavité (12), l'équipement d'amenée (42, 44, 46, 48) allant d'un premier couplage (42) dans la zone proximale (22) de l'instrument médical (20) jusqu'à une sortie (48) dans la zone distale (28) ;
un équipement d'évacuation (52, 54, 56, 58) destiné à évacuer un fluide de la cavité (12), l'équipement d'évacuation (52, 54, 56, 58) allant d'une entrée (58) dans la zone distale (28) de l'instrument médical (20) jusqu'à un deuxième couplage (52) dans la zone proximale (22) de l'instrument médical (20) ;
la résistance à l'écoulement de l'équipement d'amenée (42, 44, 46, 48) étant supérieure à la résistance à l'écoulement de l'équipement d'évacuation (52, 54, 56, 58),
dans lequel au moins
soit l'équipement d'amenée (42, 44, 46, 48) présente un moyen de résistance à l'écoulement (44) qui détermine substantiellement la résistance à l'écoulement de l'équipement d'amenée (42, 44, 46, 48),
soit l'équipement d'évacuation (52, 54, 56, 58) présente un moyen de résistance à l'écoulement (54) qui détermine substantiellement la résistance à l'écoulement de l'équipement d'évacuation (52, 54, 56, 58),
dans lequel
le moyen de résistance à l'écoulement (44 ; 54) présente un nombre fini de configurations prédéterminées en variante ayant différentes résistances à l'écoulement, et
le moyen de résistance à l'écoulement (44 ; 54) est prévu et réalisé pour fonctionner exclusivement dans les configurations prédéterminées.

2. Instrument médical (20) selon la revendication précédente, dans lequel, lors de l'utilisation prévue de l'instrument médical (20), l'équipement d'évacuation (52, 54, 56, 58) ne peut pas être fermé ou ne peut être fermé que d'une manière qui entraîne une ouverture de l'équipement d'évacuation (52, 54, 56, 58) en cas de dépassement d'une pression prédéterminée.

3. Instrument médical (20) selon l'une quelconque des revendications précédentes, dans lequel l'équipement d'évacuation (52, 54, 56, 58) comprend une soupape de sûreté qui s'ouvre en cas de dépassement d'une pression prédéterminée dans l'équipement d'évacuation.

4. Instrument médical (20) selon l'une quelconque des revendications précédentes, dans lequel la plus petite superficie de la section (45 ; 55) de l'équipement d'amenée (42, 44, 46, 48) ou de l'équipement d'évacuation (52, 54, 56, 58) se trouve au niveau du moyen de résistance à l'écoulement (44 ; 54).

5. Instrument médical (20) selon la revendication 1 et 3, dans lequel le moyen de résistance à l'écoulement (44 ; 54) présente une résistance à l'écoulement réglable.

6. Instrument médical (20) selon l'une quelconque des revendications précédentes, dans lequel le moyen de résistance à l'écoulement (44 ; 54) présente un équipement d'arrêt (72, 74, 76 ; 82, 84 ; 92) qui n'autorise que les configurations prédéterminées du moyen de résistance à l'écoulement (44 ; 54) ou qui présente des configurations prédéterminées privilégiées par rapport à d'autres configurations.

7. Instrument médical (20) selon l'une quelconque des revendications 5 et 6, dans lequel
le moyen de résistance à l'écoulement (44 ; 54) présente un composant (60 ; 90) rotatif ou pouvant être déplacé le long d'un trajet droit ou courbe, doté de plusieurs perçages traversants (61, 62, 63),
dans chaque configuration prédéterminée du moyen de résistance à l'écoulement (44 ; 54), un fluide peut s'écouler par un ou plusieurs des perçages traversants (61, 62, 63).

8. Instrument médical (20) selon la revendication précédente, dans lequel
les perçages traversants (61, 62, 63) présentent différentes sections transversales,
dans chaque configuration prédéterminée du moyen de résistance à l'écoulement (44, 54), un fluide peut s'écouler par l'un des perçages traversants (61, 62, 63).

9. Instrument médical (20) selon l'une quelconque des revendications précédentes, dans lequel
l'équipement d'évacuation (52, 54, 56, 58) présente un moyen de résistance à l'écoulement (54),
le moyen de résistance à l'écoulement (54) de l'équipement d'évacuation (52, 54, 56, 58) ne ferme l'équipement d'évacuation (52, 54, 56, 58) complètement dans aucune configuration possible ou dans aucune configuration prévue de l'instrument médical (20).

10. Instrument médical (20) selon l'une quelconque des revendications précédentes, dans lequel
à la fois l'équipement d'amenée (42, 44, 46, 48) et l'équipement d'évacuation (52, 54, 56, 58) présentent respectivement un moyen de résistance à l'écoulement (44, 54),
la résistance à l'écoulement du moyen de résistance à l'écoulement (44) de l'équipement d'amenée (42, 44, 46, 48) est supérieure à la résistance à l'écoulement du moyen de résistance à l'écoulement (54) de l'équipement d'évacuation (52, 54, 56, 58).

11. Instrument médical (20) selon la revendication précédente, dans lequel la section transversale minimale (45) du moyen de résistance à l'écoulement (44) de l'équipement d'amenée (42, 44, 46, 48) est inférieure à la section transversale minimale (55) du moyen de résistance à l'écoulement (54) de l'équipement d'évacuation (52, 54, 56, 58).
